# EUROPÄISCHE PATENTANMELDUNG

(11) **EP 1 110 506 A2**
(43) Veröffentlichungstag der Anmeldung: **27.06.2001**
(21) Anmeldenummer: 00126944.8
(22) Anmeldetag: 08.12.2000
(51) Int. Cl.: A61B 8/13

(54) **Verfahren sowie System zur Generierung von diagnostisch verwertbaren dreidimensionalen Ultraschallbilddatensätzen**

(30) Priorität: 21.12.1999 DE 19961651; 18.01.2000 DE 10001817
(71) Anmelder: EchoTech GmbH, 85399 Hallbergmoos (DE)
(72) Erfinder: Polz, Hans Dr., 85456 Wartenberg (DE)
(74) Vertreter: Graf, Helmut, Dipl.-Ing.

(57) **Zusammenfassung**

Die Erfindung bezieht sich auf ein neuartiges Vefahren sowie ein neuartiges System zur Generierung von diagnostisch verwertbaren dreidimensionalen Ultraschallbilddatensätzen

## Beschreibung

Die Erfindung bezieht sich auf ein Verfahren gemäß Oberbegriff Patentanspruch 1 sowie auf ein System zum Durchführen dieses Verfahrens gemäß Oberbegriff Patentanspruch 6.

Ein Verfahren zur Erfassung von diagnostisch verwertbaren dreidimensionalen Ultraschallbilddatensätzen ist grundsätzlich bekannt und beispielsweise in der EP 0 865 765 beschrieben. Ein wesentlicher Gesichtspunkt dieses bekannten Verfahrens ist u.a. das freie Führen des Ultraschallkopfes während der Aufnahme der Ultraschall-Einzelbilder, d.h. während der Bild- oder Datenaquisition.

Für die Gewinnung der Positions- und Orientierungsdaten wird beim bekannten Verfahren ein elektromagnetisches Sensorsystem verwendet, welches beispielsweise unter der Bezeichnung "ISOTRAK II" von der Firma POLHEMUS, One Hercules Drive, Colchester, VT 05446 angeboten wird.

Aufgabe der Erfindung ist es, ein Verfahren sowie ein System zur Generierung von diagnostisch verwertbaren dreidimensionalen Ultraschallbilddatensätzen aufzuzeigen, bei dem die Ultraschallbilder von einer Sonde, beispielsweise einer TEE-Sonde oder - Probe geliefert werden.

Zur Lösung dieser Aufgabe ist ein Verfahren entsprechend dem Patentanspruch 1 ausgebildet. Ein System ist entsprechend dem Patentanspruch 5 ausgebildet.

Weiterbildungen der Erfindung sind Gegenstand der Unteransprüche. Die Erfindung wird im folgenden anhand der Figuren an einem Ausführungsbeispiel näher erläutert. Es zeigen:

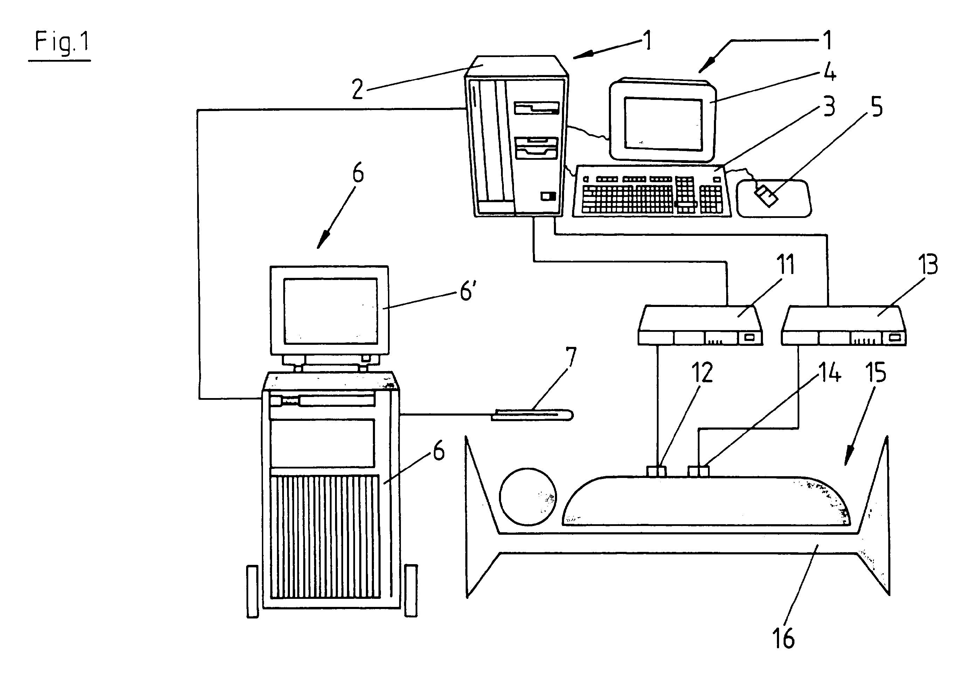

- Fig. 1: in schematischer Darstellung eine Ausführung des erfindungsgemäßen Systems zur Generierung von diagnostisch verwertbaren dreidimensionalen Datensätzen für eine tomographische Bildabfassung eines zu untersuchenden Volumens, unter Verwendung einer einführbaren Ultraschall-Sonde;

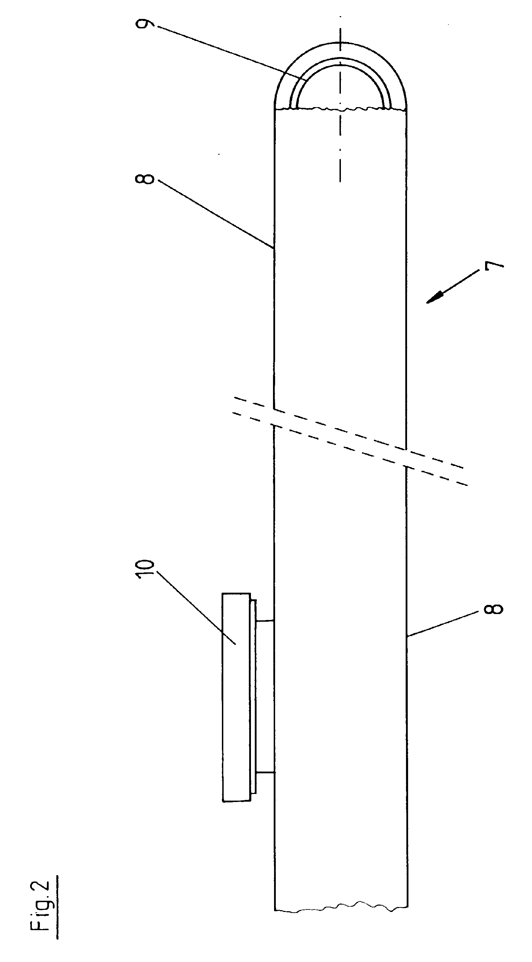

- Fig. 2: in vereinfachter Einzeldarstellung die Sonde;
- Fig. 3: in schematischer Darstellung die jeweils im Bildverarbeitungssystem abgelegten Einzelbilder, zusammen mit graphischen Darstellungen des EKG-Zyklus und des Respirations-Zyklus eines Patienten.

In den Figuren ist 1 ein Bildverarbeitungssystem, welches im wesentlichen aus dem Rechner 2 und aus den an diesem angeschlossenen peripheren Geräten, wie Tastatur 3, Bildschirm 4, Maussteuerung 5 usw. besteht.

Dem Bildverarbeitungssystem 1 ist zugeordnet ein Ultraschallgerät 6 mit einer Ultraschall-Sonde 7, die bei der dargestellten Ausführungsform eine TEE-Sonde oder - Probe, d.h. eine Sonde für die Transösophageale Echokardiographie ist.

Sonden dieser Art sind dem Fachmann bekannt. Sie bestehen im wesentlichen aus einem schlauchartigen Sonden- oder Probenkörper 8, in dem die Funktionselemente der Sonde aufgenommen sind und der an einem Ende die Sondenspitze bildet, an welcher innerhalb des Sondenkörpers 8 eine Ultraschall-Kristall-Anordnung 9 untergebracht ist, die um die Sondenachsen dreh- oder schwenkbar ist, und zwar in nicht dargestellter Weise über eine Mechanik, beispielsweise über eine im Sondenkörper 8 untergebrachte biegsame Welle mittels eines Rades oder Stellgliedes 10. Dieses ist mit dem erforderlichen Abstand von der Sondenspitze am Sondenkörper 8 vorgesehen. Die Ultraschall-Anordnung 9 liefert Bilder von Bildebenen oder Schnitten, die radial zur Sondenachse orientiert sind.

Die Sonde 7 ist über eine mehradrige Signalleitung an das Ultraschallgerät 6 angeschlossen, über die dann nicht nur die Bildinformationen an das Ultraschall-Gerät übertragen werden, sondern auch eine die jeweilige Drehstellung des Ultraschall-Kopfes 9 definierende Information, die von einer in der Sonde 7 untergebrachten Elektronik, beispielsweise mit Winkelgeber geliefert wird. Das Ultraschallgerät 6 generiert aus den von der Sonde 7 gelieferten Bilddaten Ultraschall-Einzelbilder deren Bildebene abhängig ist u.a. von der Winkelstellung der Ultraschall-Anordnung 9. Die Einzelbilder können dann auf dem Bildschirm 6' des Ultraschallgeräte s dargestellt werden, und zwar zusammen mit einer in das jeweilige Bild eingeblendeten Angabe über die Winkelstellung der Ultraschall-Anordnung 9.

Bei der dargestellten Ausführungsform sind dem Bildverarbeitungssystem weiterhin noch ein EKG-Gerät 11 mit zugehörigen Elektroden 12 und ein Gerät 13 zur Registrierung der Atemlage (Respirationszyklus) mit zugehörigen Sonden 14 zugeordnet.

Um aus den von dem Ultraschallgerät 6 gelieferten Einzelbildern einen dreidimensionalen, diagnostisch verwertbaren Datensatz zu erstellen, werden die Einzelbilder von dem an einen geeigneten Ausgang des Ultraschallgerätes 6 angeschlossenen Rechner 2 des Bildverarbeitungssystems 1 erfaßt. Liegen die Einzelbilder in analoger Form vor, so erfolgt mit geeigneten Mitteln, beispielsweise mittels Framegrabber eine Digitalisierung dieser analogen Bilder bzw. Videobilder. Liegen die Ultraschalleinzelbilder bereits in digitaler Form vor, so können diese vom Rechner ohne Umwandlung erfaßt und verarbeitet werden. Der Bildinhalt der Einzelbilder enthält auch, wie oben bereits ausgeführt wurde, die Positionsinformation, d.h. die Information über die Winkelstellung der Ultraschall-Anordnung 9.

Mit einer geeigneten Software wird diese Positionsinformation erkannt und extrahiert. Bei analogen, vom Ultraschallgerät gelieferten Einzelbildern erfolgt diese Erkennung und Extraktion der Positionsinformation mit Hilfe eines Verfahrens bzw. einer Software zur Mustererkennung (z.B. OCR = Optical Character Recognition).

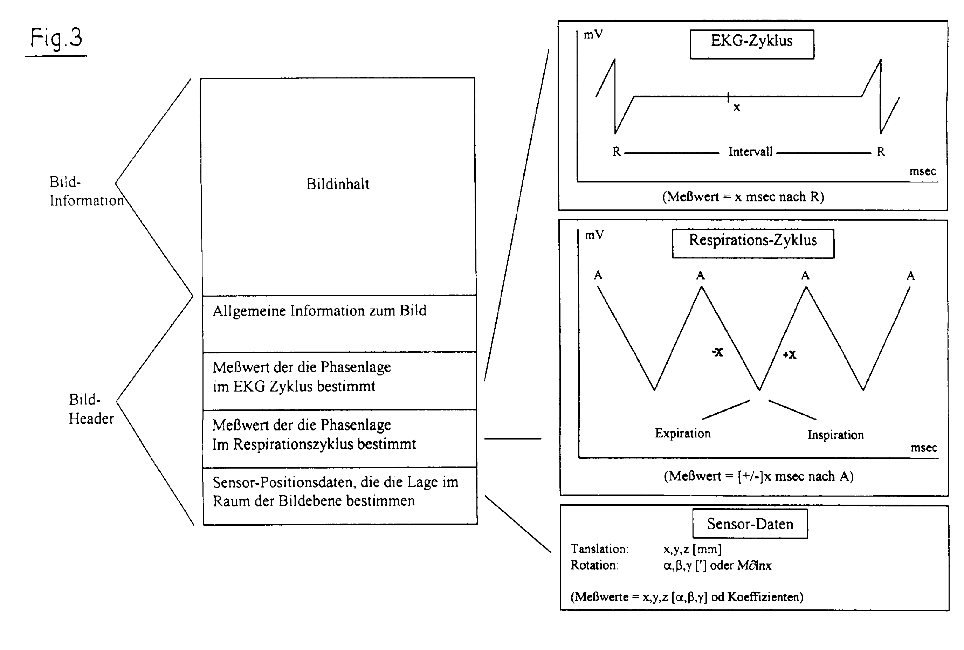

Im Rechner wird dann zu jedem Ultraschall-Einzelbild auch die zugehörige Positionsinformation in einem diesem Einzelbild zugeordneten Datensatz abgespeichert, und zwar entsprechend der Figur 3 in einem Bildhaeder dieses Datensatzes, wobei der Bildhaeder bei der dargestellten Ausführungsform auch noch Werte des EKG-Gerätes 11, z.B. einen Meßwert, der die Phasenlage des jeweiligen Einzelbildes in bezug auf den EKG-Zyklus bestimmt, und/oder z.B. einen Meßwert des Gerätes 13, der beispielsweise Phasenlage des Einzelbildes in bezug auf den Respirationszyklus bestimmt.

Die Sonde 7, die beispielsweise die Aufnahme von Bildsequenzen in unterschiedlichen Schnittebenen des menschlichen Herzens ermöglicht, wird hierfür in die Speiseröhre des Patienten 15 eingeführt.

Die Zuordnung der Postionsinformation zu dem jeweiligen Ultraschall-Einzelbild, d.h. das Abspeichern dieser Information sowie weitere Informationen im Bild-Haeder kann Online, d.h. zeitgleich mit der Aufnahme der Einzelbilder oder aber nach der Bilderfassung in einem ersten Schritt einer Nachbearbeitung erfolgen.

Insbesondere dann, wenn die Positionsinformation "online" verarbeitet wird, kann diese Information auch dazu verwendet werden, die Bilderfassung bzw. den Bildeinzug durch den Rechner 2 zu steuern, d.h. ein Ultraschall-Einzelbild wird nur dann erfaßt, wenn seit dem letzten, erfaßten Einzelbild ein vorbestimmtes oder vorgegebenes Winkelinkrement der Drehstellung der Ultraschall-Anordnung 9 zurückgelegt wurde.

Durch das EKG-Gerät und das Gerät 13 besteht die Möglichkeit, dynamische, aber auch statische dreidimensionale Datensätze zu generieren, und zwar in bezug auf den Herzzyklus bzw. die Atmung des Patienten 15. Insbesondere besteht auch die Möglichkeit der EKG-Triggerung und eines Atem-Gating.

Die im Rechner 2 abgespeicherten Bilddaten mit zugehörigem Bildhaeder bilden einen Rohdatensatz, aus dem dann beispielsweise durch ein Transformation- und/oder Interpolationsverfahren der diagnostisch verwertbare dreidimensionale Datensatz in einem einheitlichen Bezugs- oder Koordinatensystem erstellt wird. Hierfür wird dann beispielsweise in der EP 0 865 765 detailliert beschriebenen Weise vorgegangen.

Sind bei dem verwendeten Ultraschall-Gerät bzw. der zugehörigen Sonde zusätzlich zu den tatsächlich gelieferten und angezeigten Positionsinformationen geometrische Annahmen getroffen, z.B. Rotation der Ultraschall-Anordnung 9 um eine in ihrer Lage definierte Achse, beispielsweise um eine durch die Mitte des Schallkopfes 9 verlaufende Achse, so sind auch diese Annahmen in dem jeweiligen Bildhaeder abgelegt, so daß diese Daten bei der Erstellung des dreidimensionalen Datensatzes berücksichtigt werden.

Die von der Sonde 7 gelieferte Positionsinformation, d.h. Information über die Winkelstellung der Ultraschall-Anordnung 9 ist beispielsweise eine numerische und/oder graphische Information.

Die Erfindung wurde voranstehend an einem Ausführungsbeispiel beschrieben. Es versteht sich, daß Änderungen sowie Abwandlungen möglich sind, ohne daß der der Erfindung zugrundeliegende Erfindungsgedanke verlassen wird.

Enthalten die vom Ultraschall-Gerät 6 gelieferten Einzelbilder die Positionsinformation bereits in einem Bild-Haeder, so kann diese Information direkt für die Erstellung des dreidimensionalen Volumendatensatzes verwendet werden.

Weiterhin ist es möglich, daß der Rechner 2 Bestandteil des Ultraschallgerätes 6 ist. In diesem Fall ist es dann möglich, daß das entsprechende Programm zur Generierung der Ultraschallbilder aus den Ultraschallsignalen der Sonde 7 und der Positionsinformationen diese Bilddaten und Positionsinformationen über eine geeignete Schnittstelle, z.B. über OLE-Schnittstelle oder shared memory file oder shared memory Mechanismen an das Programm zur Generierung des dreidimensionalen Volumendatensatzes weitergibt.

## Patentansprüche

1. Verfahren zur Generierung eines diagnostisch verwertbaren dreidimensionalen Bilddatensatzes unter Verwendung eines Ultraschallgerätes mit Ultraschall-Anordnung (9) zur Erzeugung einer Sequenz von Ultraschallbildern in unterschiedlichen Ebenen des zu untersuchenden Volumens, unter Verwendung einer Bildverarbeitungseinrichtung (1), der die Ultraschalleinzelbilder zugeführt werden sowie unter Verwendung von Mitteln zur Erzeugung von die Lage der Ultraschall-Anordnung (9) definierenden Positionsinformationen, die ebenfalls an die Bildverarbeitungseinrichtung (1) übertragen werden, die aus den Bilddaten der Ultraschall-Einzelbilder und den Positionsinformationen einen dreidimensionalen, das untersuchte Volumen tomographisch erfassenden Volumen-Datensatz generiert, dadurch gekennzeichnet, daß die wenigstens eine Ultraschall-Anordnung (9) Bestandteil einer Sonde (7), vorzugsweise Bestandteil einer TEE-Sonde oder -Probe ist.

2. Verfahren nach Anspruch 1, dadurch gekennzeichnet, daß die wenigstens eine Ultraschall-Anordnung (9) Bestandteil einer in das zu untersuchende Volumen einführbaren Sonde (7) ist.

3. Verfahren nach Anspruch 1 oder 2, dadurch gekennzeichnet, daß das Ultraschallgerät aus den von der Sonde (7) gelieferten Bilddaten Einzelbilder generiert, die im jeweiligen Bild oder in den jeweiligen Bilddaten die Positionsinformation enthält, und daß diese Information in der Bildverarbeitungseinrichtung extrahiert und zusammen mit den Bilddaten, vorzugsweise in einem Bildhaeder der Bilddaten gespeichert wird.

4. Verfahren nach einem der vorhergehenden Ansprüche, dadurch gekennzeichnet, daß die Positionsinformation von der Datenverarbeitungseinrichtung (1) aus einem von dem Ultraschallgerät (6) gebildeten Bildhaeder der Einzelbilder übernommen wird.

5. Verfahren nach einem der vorhergehenden Ansprüche, dadurch gekennzeichnet, daß bei in das Ultraschallgerät (6) integrierter Bildverarbeitungseinrichtung (1) die von einem Rechner aus den Daten der Sonde (7) ermittelten Positionsinformationen direkt für die Generierung des dreidimensionalen Bilddatensatzes übernommen werden.

6. System zur Generierung eines diagnostisch verwertbaren dreidimensionalen Bilddatensatzes unter Verwendung eines Ultraschallgerätes mit Ultraschall-Anordnung (9) zur Erzeugung einer Sequenz von Ultraschallbildern in unterschiedlichen Ebenen des zu untersuchenden Volumens, unter Verwendung einer Bildverarbeitungseinrichtung (1), der die Ultraschalleinzelbilder zugeführt werden sowie unter Verwendung von Mitteln zur Erzeugung von die Lage der Ultraschall-Anordnung (9) definierenden Positionsinformationen, die ebenfalls an die Bildverarbeitungseinrichtung (1) übertragen werden, die aus den Bilddaten der Ultraschall-Einzelbilder und den Positionsinformationen einen dreidimensionalen, das untersuchte Volumen tomographisch erfassenden Volumen-Datensatz generiert, dadurch gekennzeichnet, daß die wenigstens eine Ultraschall-Anordnung (9) Bestandteil einer in das zu untersuchende Volumen einführbaren Sonde (7), vorzugsweise Bestandteil einer TEE-Sonde oder -Probe ist.

7. System nach Anspruch 6, dadurch gekennzeichnet,
daß die wenigstens eine Ultraschall-Anordnung (9) Bestandteil einer in das zu untersuchende Volumen einführbaren Sonde (7) ist, und/oder

8. System nach Anspruch 6 oder 7, dadurch gekennzeichnet, daß das Ultraschallgerät aus den von der Sonde (7) gelieferten Bilddaten Einzelbilder generiert, die im jeweiligen Bild oder in den jeweiligen Bilddaten die Positionsinformation enthält, und daß diese Information in der Bildverarbeitungseinrichtung extrahiert und zusammen mit den Bilddaten, vorzugsweise in einem Bildhaeder der Bilddaten gespeichert wird.

9. System nach einem der vorhergehenden Ansprüche, dadurch gekennzeichnet, daß die Positionsinformation von der Datenverarbeitungseinrichtung (1) aus einem von dem Ultraschallgerät (6) gebildeten Bildhaeder der Einzelbilder übernommen wird.

10. System nach einem der vorhergehenden Ansprüche, dadurch gekennzeichnet, daß bei in das Ultraschallgerät (6) integrierter Bildverarbeitungseinrichtung (1) die von einem Rechner aus den Daten der Sonde (7) ermittelten Positionsinformationen direkt für die Generierung des dreidimensionalen Bilddatensatzes übernommen werden.
